(19) Europäisches Patentamt
European Patent Office
Office européen des brevets

(11) Publication number: **0 487 188 A1**

# (12) EUROPEAN PATENT APPLICATION

(21) Application number: **91307391.2**

(51) Int. Cl.[5]: **C08G 14/06**

(22) Date of filing: **12.08.91**

(30) Priority: **19.11.90 US 615103**
**19.11.90 US 615104**

(43) Date of publication of application:
**27.05.92 Bulletin 92/22**

(84) Designated Contracting States:
**DE FR GB IT**

(71) Applicant: **TEXACO CHEMICAL COMPANY**
**3040 Post Oak Boulevard**
**Houston, Texas 77056(US)**

(72) Inventor: **Speranza, George Phillip**
**2800 Silverleaf**
**Austin, TX 78757(US)**
Inventor: **Lin, Jiang-Jen**
**15031 Rock Knoll**
**Houston, TX 77083(US)**

(74) Representative: **Brock, Peter William et al**
**UROUHART-DYKES & LORD 91 Wimpole Street**
**London W1M 8AH(GB)**

## (54) Alkyl phenol Mannich condensates.

(57) Mannich condensation products having the formula

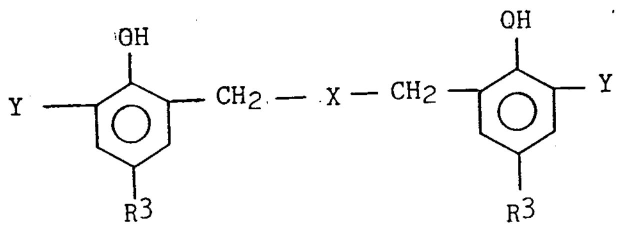

constitute a novel class of curing agents for epoxy resins.

In the above formula

X is a group of the formula

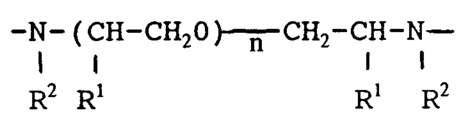

Y is hydrogen or a group of the formula

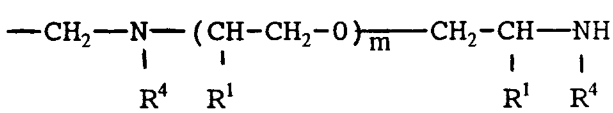

m and n are each 1-6,

$R^1$ is hydrogen or methyl

EP 0 487 188 A1

$R^2$ is hydrogen, $C_1$-$C_4$ alkyl or cyanoethyl,
$R^3$ is $C_1$-$C_{15}$ alkyl, and
$R^4$ is hydrogen, $C_1$-$C_4$ alkyl or cyanoethyl, with the proviso that when $R^2$ is not hydrogen, $R^4$ is also not hydrogen.

This invention relates to Mannich condensates prepared from an alkyl phenol, formaldehyde and polyoxyalkylene diamines.

More particularly, this invention relates to initial Mannich condensates prepared by reacting an alkyl phenol with formaldehyde and a first primary polyoxyalkylene diamine to provide intermediate alkyl phenol Mannich condensates, which are further reacted with formaldehyde and a second polyoxyalkylene diamine, in order to provide the final Mannich condensates.

Both intermediate Mannich condensates and the final Mannich condensates are useful as epoxy resin curing agents.

The Mannich reaction is a well-known reaction which has been extensively reviewed in the literature; see, for example, "The Mannich Reaction ", Org. Reactions 1, 303 (1942), and "Advances in the Chemistry of Mannich Bases", "Methods in Synthetic Organic Chemistry-Synthesis", Academic Press, pp. 703-775, 1973.

US-A-4485195 discloses Mannich condensates prepared by reacting an alkyl phenol with formaldehyde, diethanolamine and a minor amount of melamine, to provide products which can be alkoxylated for use in the preparation of fire retardant, rigid polyurethane foams.

Other Mannich condensates prepared by reacting alkyl phenols with formaldehyde, alkanolamines and melamine are disclosed in US-A-4487852, -4489178, and -4500655.

US-A-4736011 discloses Mannich condensates, which are useful as curing agents for epoxy resins, and are prepared by the reaction of an imidazole with formaldehyde and a polyoxyalkylene polyamine.

US-A-3734965 discloses phenolic resins prepared by condensing a polyoxypropylenepolyamine with phenol and an aldehyde.

US-A-4714750 describe the Mannich condensates prepared from 2,6-di-t-butylphenol, formaldehyde and a polyoxyalkyleneamine, i.e:

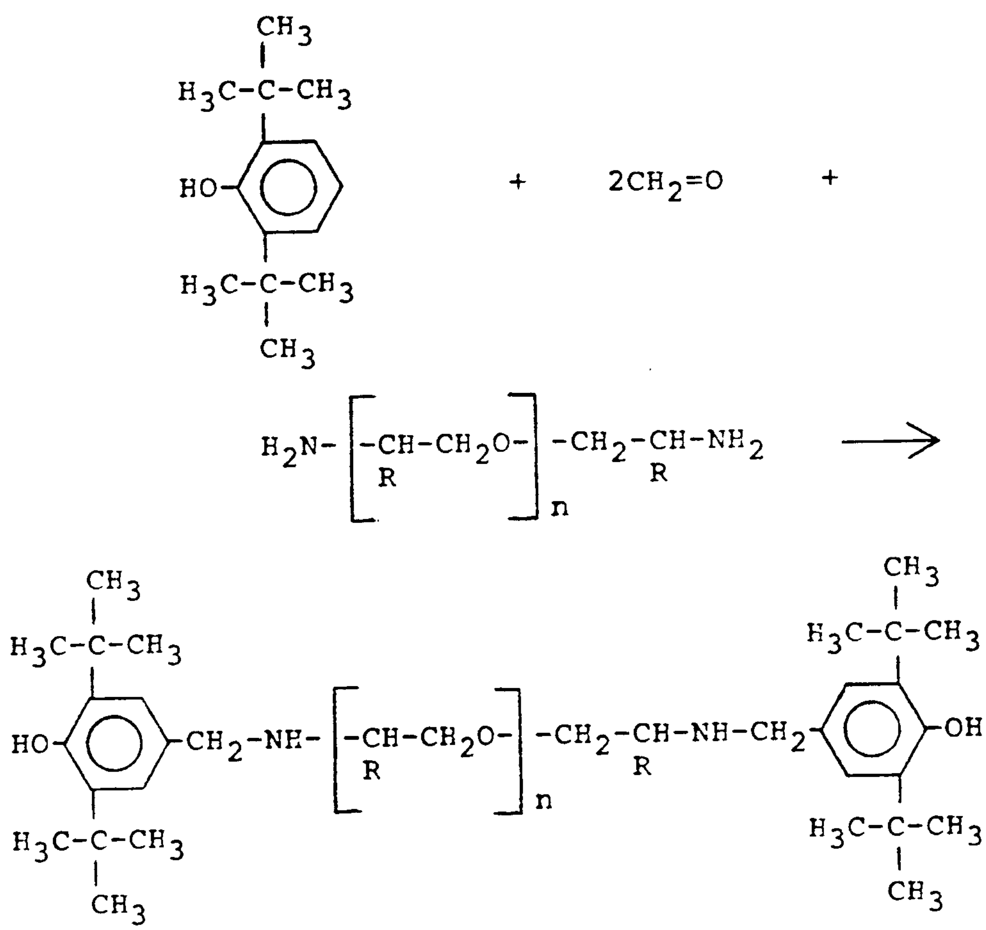

where: R = H or $CH_3$, and n = 1 to 20.

In one embodiment, the present invention provides Mannich condensation products having the formula

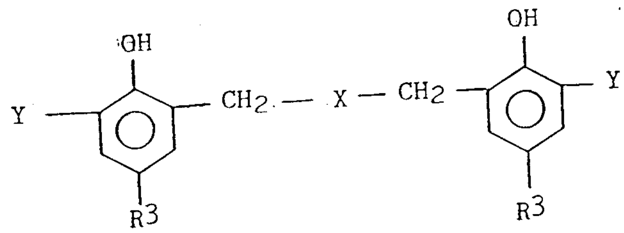

I

wherein
X is a group of the formula

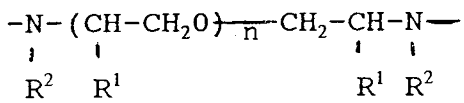

II

Y is hydrogen or a group of the formula

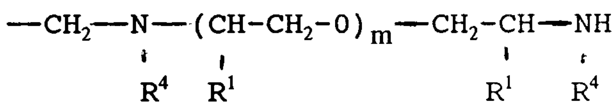

III

m and n are each 1-6,
$R^1$ is hydrogen or methyl
$R^2$ is hydrogen, $C_1$-$C_4$ alkyl or cyanoethyl,
$R^3$ is $C_1$-$C_{15}$ alkyl, and
$R^4$ is hydrogen, $C_1$-$C_4$ alkyl or cyanoethyl, with the proviso that when $R^2$ is not hydrogen, $R^4$ is also not hydrogen.

According to another embodiment, this invention provides Mannich condensation products having the formula

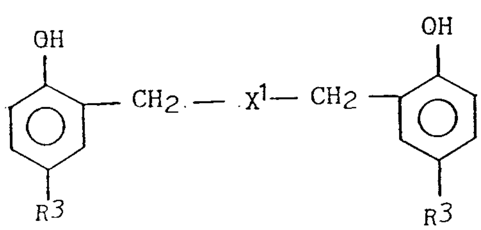

IV

wherein
$X^1$ is a group of the formula

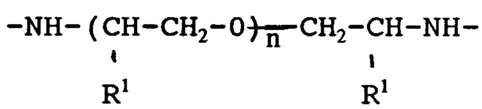

V

and $R^1$, $R^3$ and n have the meanings given above.

A further embodiment of this invention provides Mannich condensation products having the formula

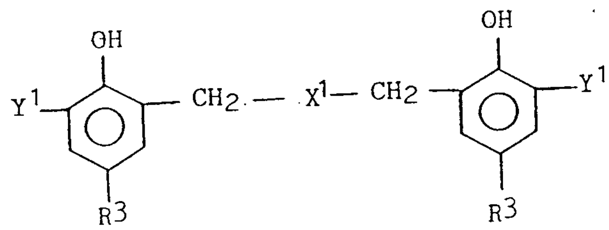

VI

wherein
$X^1$ is a group of the formula V as defined above.
$Y^1$ is a group of the formula

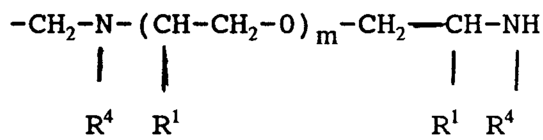

VII

and $R^1$, $R^3$, $R^4$, m and n have the meanings given above.

A further embodiment of the invention provides Mannich condensation products having the formula

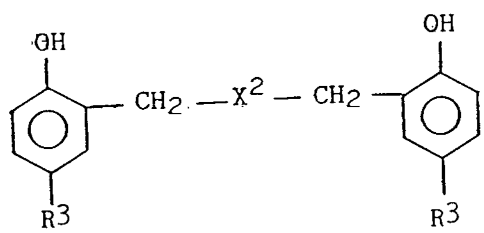

VIII

wherein
$X^2$ is a group of the formula II in which
$R^1$, $R^3$ and n have the meanings given in respect of formula II and $R^2$ is $C_1$-$C_4$ alkyl or cyanoethyl.

A further embodiment of the invention provides Mannich condensation products having the formula

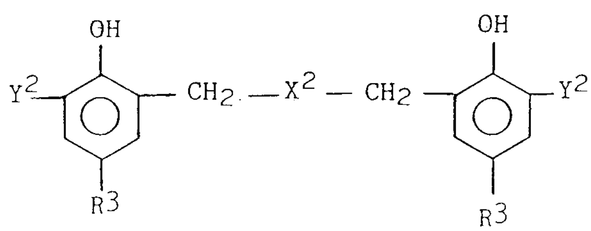

IX

wherein
$X^2$ has the meaning given above, and
$Y^2$ is a group of the formula III in which
$R^1$, $R^3$, m and n have the meanings given in respect of formula III, and $R^2$ and $R^4$ are $C_1$-$C_4$ alkyl or cyanoethyl.

The invention further provides a method for the preparation of a compound of formula I in which each Y is H by reacting a phenol of the formula

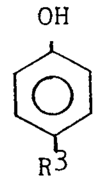

X

with formaldehyde and a polyoxyalkylene diamine of the formula

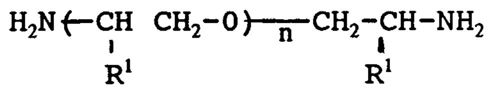

XI

wherein $R^1$, $R^3$ and n have the meanings given in respect of formula I.

A further embodiment provides a method for the preparation of a compound of formula VI by reacting a compound of the formula IV with formaldehyde and a polyoxyalkylene diamine of the formula

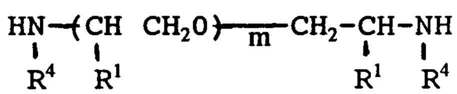

XII

wherein $R^1$, $R^4$ and m have the meanings given in respect of formula I.

In yet another embodiment, a compound of formula VIII is obtained by reacting a phenol of the formula X with formaldehyde and a polyoxyalkylene diamine of the formula XII.

The compound of formula VIII can be converted into a compound of formula IX by reacting it with formaldehyde and a polyoxyalkylene diamine of the formula XII, which can be the same as, or different from the compound of formula XII used to produce the compound of formula VIII.

The invention also relates to the use of a Mannich condensation product as defined above as a curing agent for epoxy resins.

It has been further discovered that the "paired" initial alkyl phenol Mannich condensates of formula IV or VIII (i.e., Mannich condensates containing two phenolic rings derived from the alkyl phenol, that are separated by a diamine group, derived from the formaldehyde and the first polyoxyalkylenediamine) are liquid at ambient temperatures.

The starting materials for the present invention are formaldehyde, a phenol of formula; X having a $C_1$-$C_{15}$ alkyl group in the para position, a defined first class of polyoxyalkylene diamines of formula XI or XII and a defined second class of polyoxyalkylene diamines of formula XII.

The First Class of Polyoxyalkylene Diamine Starting materials

The first class of polyoxyalkylene diamine starting materials for the present invention are polyoxyethylene or polyoxypropylene diamines having the formula XI or formula XII in which $R^4$ is $C_1$-$C_4$ alkyl or cyanoethyl.

Examples of polyoxyethylene diamine starting materials include products made by Texaco Chemical Company under the tradenames "Jeffamine EDR-148" (wherein n is about 2) and "Jeffamine EDR-192" (wherein n has a value of 3).

Another example of a group of polyoxyalkylene diamine starting materials are polyoxypropylene diamines having the formula:

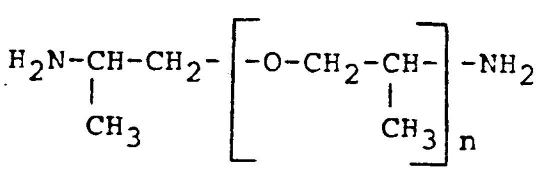

XIII

wherein n is 1-6

Examples of products of this formula include products sold by the Texaco Chemical Company under the tradename "Jeffamine D" including, for example, "Jeffamine D-230" (wherein n is 2-3), and "Jeffamine D-400" (wherein n is 5-6).

N-alkyl derivatives of the polyoxypropylene diamines, wherein R' is a $C_1$-$C_4$ alkyl group that may be used as starting materials include, for example, the N-isopropyl derivatives having the formula:

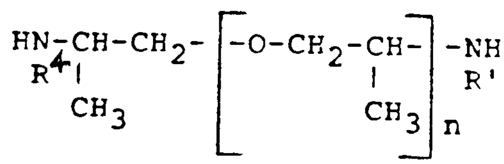

XIV

wherein:

$R^4$ is an isopropyl group, and n is 1-6. Such compounds of formula XIV can be prepared by the method disclosed in US-A-4927912.

Cyanoethyl derivatives of polyoxypropylene diamines, wherein $R^4$ is cyanoethyl that may be used as starting materials include, for example, a cyanoethyl derivative of a polyoxypropylene diamine having the formula:

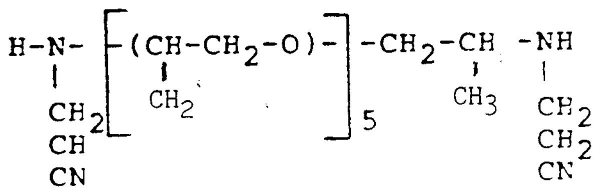

XV

## The Formaldehyde

Formaldehyde may be employed in any of its conventional forms. Thus, it may be, and preferably is, used in the form of an aqueous solution of formaldehyde such as "formalin" and may also be used in "inhibited" methanol solution as paraformaldehyde or as trioxane .

## The Phenol Starting Material

The phenol should be a para alkyl phenol having the formula X wherein the alkyl group $R^3$ has 1 to 15 carbon atoms.

Representative examples of suitable phenols include p-methyl phenol, p-ethyl phenol, p-propyl phenol, p-isopropyl phenol, the p-substituted n-butyl-, isobutyl- and tertiary butyl phenols, p-amyl phenol, p-decyl phenol, p-nonyl phenol, p-dodecyl phenol, p-pentadecyl phenol, etc. The phenols containing the larger alkyl groups are frequently prepared by reacting phenol with a dimer, trimer, tetramer or pentamer of propylene.

## The Second Class of Polyoxyalkylene Diamine Starting Materials

The second class of polyoxyalkylene diamine starting materials to be used in accordance with the present invention, which may be the same as or different from the first class of polyoxyalkylene diamine starting materials have the formula XII as defined above. It should be noted, however, that when $R^2$ is not hydrogen, $R^4$ is also not hydrogen. The second diamine should not be stronger than the first diamine. In other respects reference should be made for suitable diamines to the information set out above in respect of the first class of diamines.

## Preparation of the Initial Mannich Condensates

In accordance with the present invention, the initial Mannich condensate is prepared by reacting the para- alkyl phenol of formula X with formaldehyde and a first polyoxyalkylene diamine in the mole ratio of

about 2:2:1 under Mannich reaction conditions, including a temperature 80 to 150° for a period of 2 to 8 hours.

Pressure is not critical. The reaction is suitably conducted at atmospheric pressure, although lower or higher pressures, such as subatmospheric pressures or superatmospheric pressures of several atmospheres, may be used.

There are, however, certain limitations to the method of synthesis of paired alkyl phenol Mannich condensates as disclosed herein and the order of the addition of the reactants is very important.

For example, although the preparation goes well for bis-phenols of formula IV or VIII, we have surprisingly discovered that the second step is very critical, and that unexpected and unwanted results are obtained unless the diamine is correctly chosen having regard to the diamine used in the first step.

For example, when both $R^1$ and $R^4$ are hydrogen, the synthesis of the unique compounds of the present invention is performed with comparative ease. Thus, in Example 4 (see below) the reaction proceeded as follows:

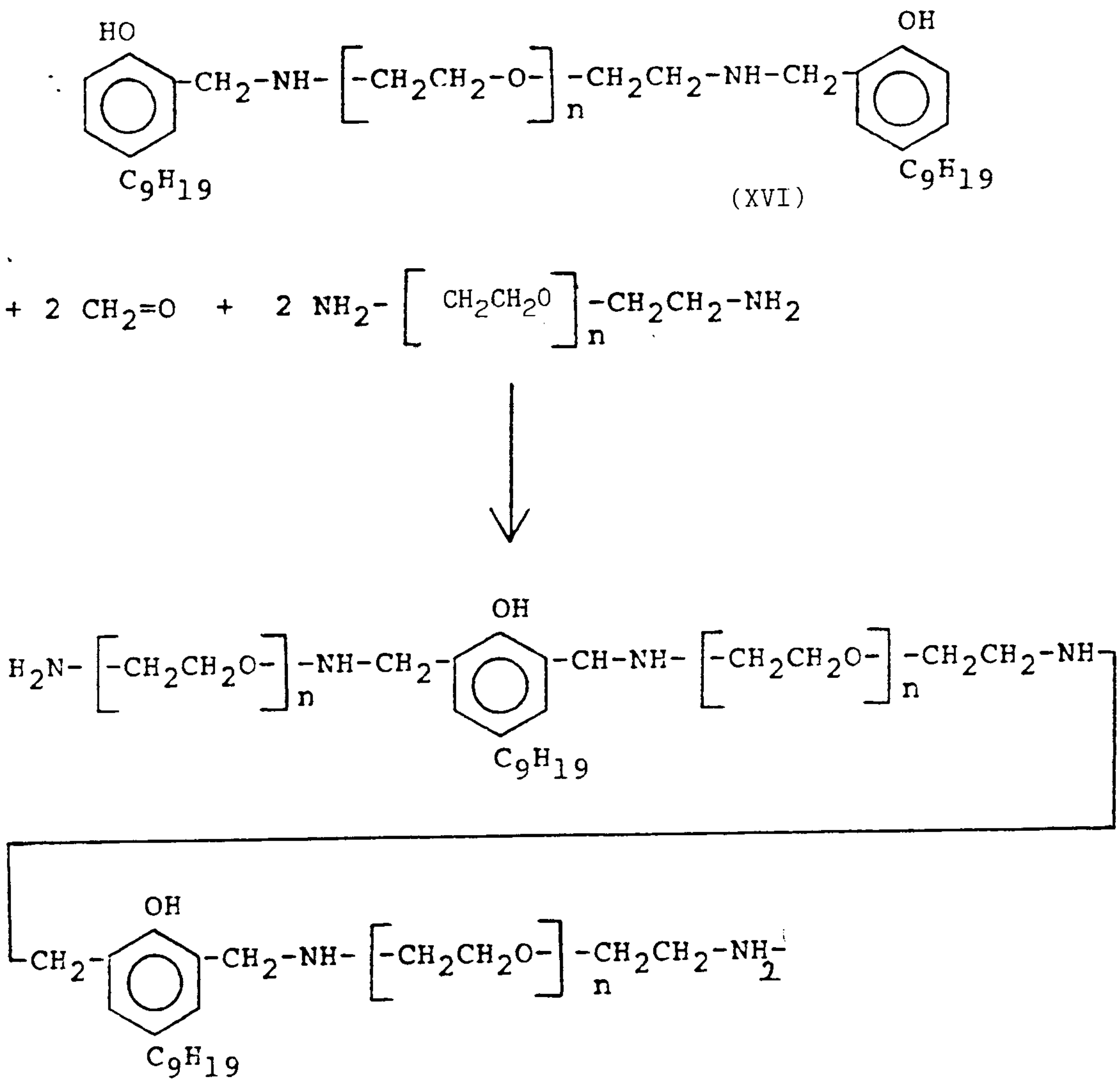

(XVI)

However, if the bisphenol of formula XVI above, is reacted with a much stronger amine, such as diethylenetriamine, a significant "scrambling" takes place so that the reaction gives products such as:

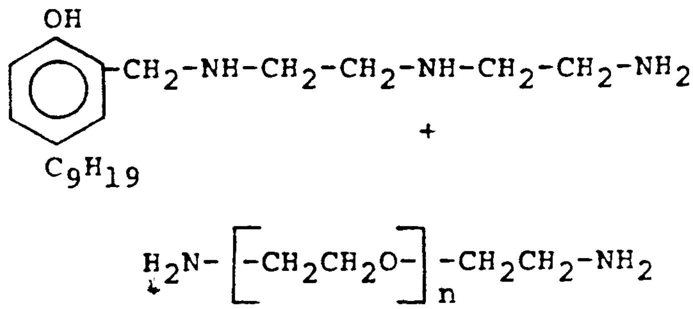

In a similar manner, if the bis-phenol is made from a dialkylpolyoxyalkylene diamine, the dialkylpolyoxyalkylene diamines are replaced with the unalkylated amine reactant. For example:

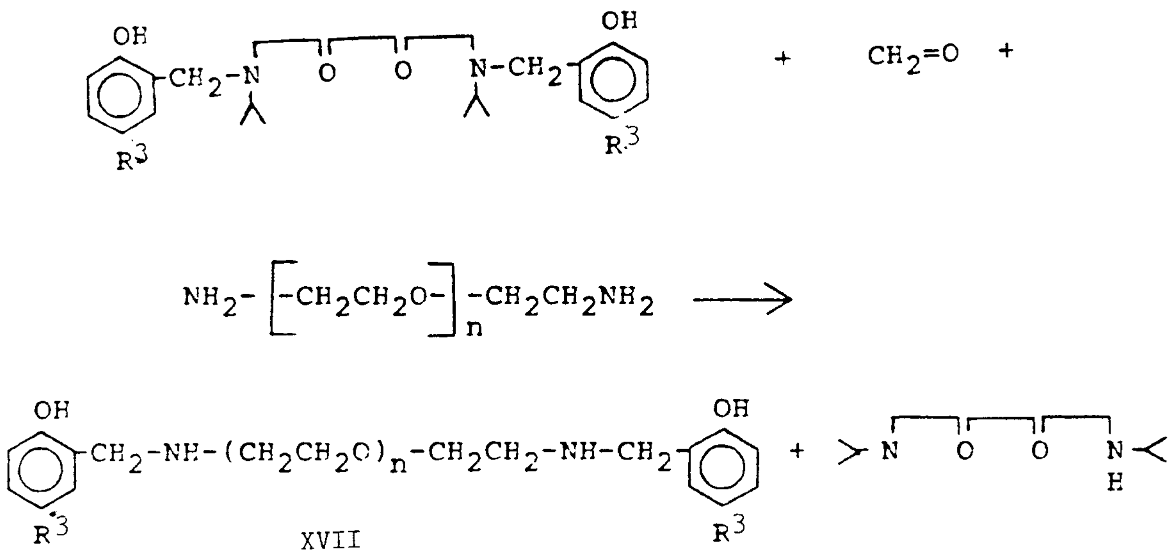

XVII

It is possible to mix the two disecondary amines, but the reactions take much longer to go to completion. For example, when the product of Formula XVII is allowed to react with a bis-cyanoethyl ether, about 80% of the ortho groups react under the usual conditions used in the other examples, i.e.:

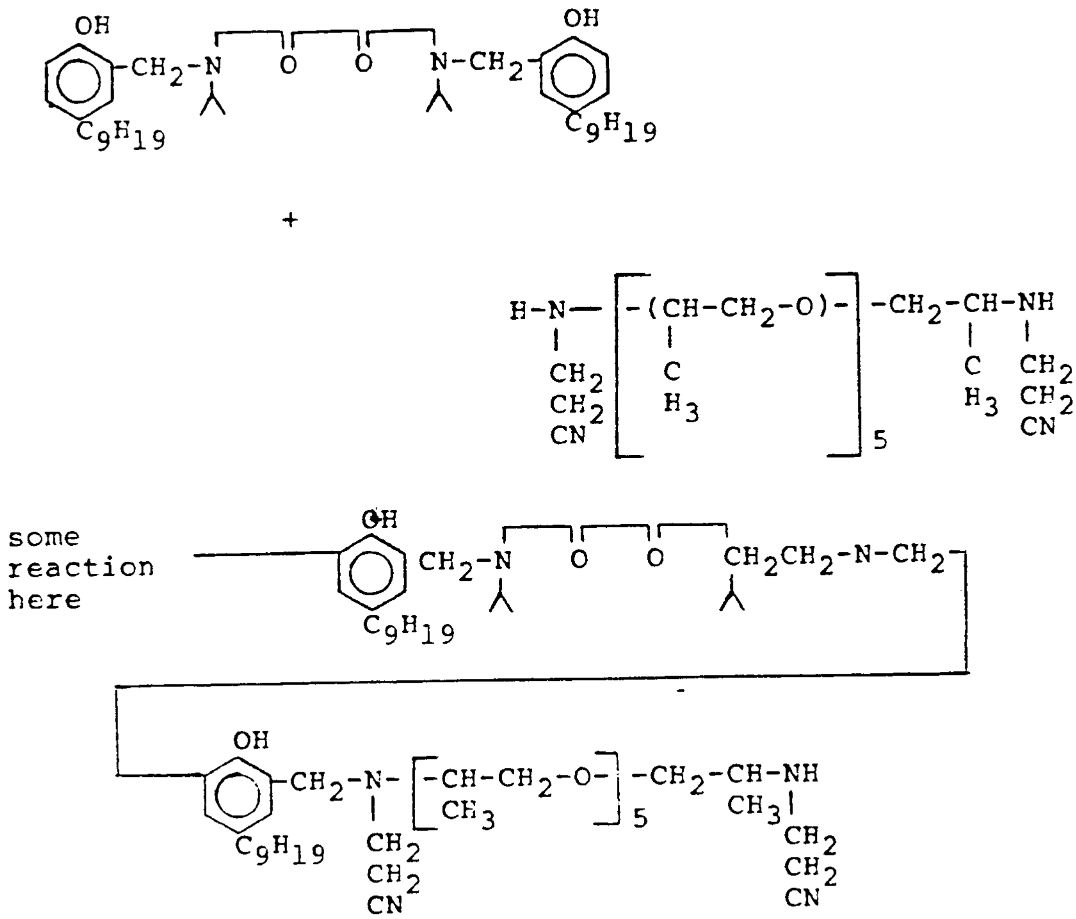


Utility of Mannich Condensates as Epoxy Curing Agents

The Mannich condensates of the present invention are useful as curing agents for 1,2-epoxy resins.

It is known to use amines, such as aliphatic or aromatic amines, for curing 1,2-epoxy resins as shown, for example, in US-A-4139524 and -4162353, and in "Handbook of Epoxy Resins" by H. Lee and K. Neville, McGraw-Hill Book Company, 1967.

Generally the vicinal epoxide compositions that can be cured using the curing agents of this invention are organic materials having an average of more than one reactive 1,-epoxide group. These polyepoxide materials can be monomeric or polymeric, saturated or unsaturated, aliphatic, cycloaliphatic, aromatic or heterocyclic, and may be substituted, if desired, with other substituents besides the epoxy groups, e.g., hydroxyl groups, ether radicals, or halogenated phenyl groups.

The most widely used epoxy resins are diglycidyl ethers of bisphenol A:

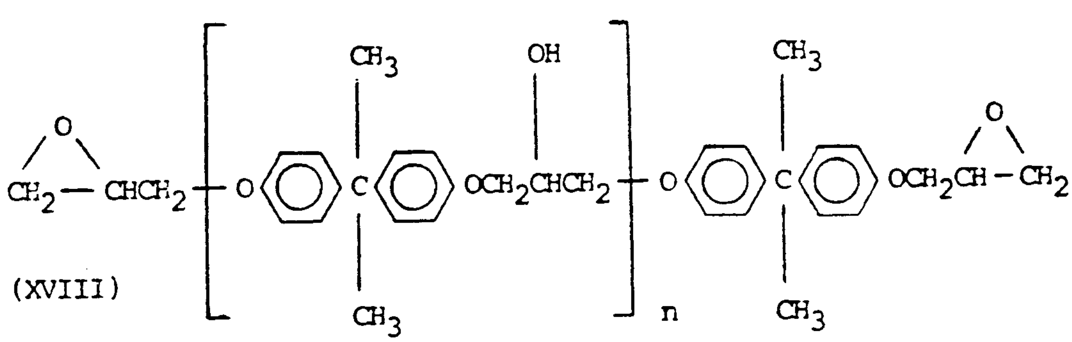

(XVIII)

where n is 10 to 20.

However, these epoxides are only representative of the broader class of epoxides that are useful in

making epoxy resins.

A widely used class of polyepoxides that can be cured includes the resinous epoxy polyethers obtained by reacting an epihalohydrin, such as epichlorohydrin, with either a polyhydric phenol or a polyhydric alcohol. An illustrative, but by no means exhaustive, listing of suitable dihydric phenols includes 4,4'-isopropylidene bisphenol, 2,4'-dihydroxydiphenylethylmethane, 3,3'-dihydroxydiphenyldiethylmethane, 3,4'-dihydroxydiphenylmethylpropylmethane, 2,3'-dihydroxydiphenylethylphenylmethane, 4,4'-dihydroxydiphenylmethane, 4,4'-dihydroxydiphenylbutylphenylmethane, 2,2'-dihydroxydiphenylditolylmethane, and 4,4'-dihydroxydiphenyltolyl-methyl-methane. Other polyhydric phenols which may also be co-reacted with an epihalohydrin to provide these epoxy polyethers are such compounds as resorcinol, hydroquinone, and substituted hydroquinones, e.g., tertbutylhydroquinone.

Among the polyhydric alcohols that can be coreacted with an epihalohydrin to provide the resinous epoxy polyethers are such compounds as ethylene glycol, propylene glycol, butylene glycols pentane diols, bis(4-hydroxycyclohexyl)dimethylmethane, 1,4-dimethylolbenzene, glycerol, 1,2,6-hexanetriol, trimethylolpropane, mannitol, sorbitol, erythritol, pentaerythritol, their dimers, trimers and higher polymers, e.g. polyethylene glycols, polypropylene glycols, triglycerol and dipentaerythritol, polyallyl alcohol, polyhydric thioethers, such as 2,2', 3,3'-tetrahydroxydipropylsulfide, mercapto alcohols such as α-monothioglycerol and α, α'-dithioglycerol, polyhydric alcohol partial esters, such as monostearin, and pentaerythritol monoacetate, and halogenated polyhydric alcohols such as the monochlorohydrins of glycerol, sorbitol and pentaerythritol.

Another class of polymeric polyepoxides that can be cured by means of the above-described curing agents includes the epoxy novolac resins obtained by reacting preferably in the presence of basic catalyst such as sodium or potassium hydroxide, an epihalohydrin, such as epichlorohydrin, with the resinous condensate of an aldehyde, e.g., formaldehyde, and either a monohydric phenol, e.g., phenol itself, or a polyhydric phenol. Further details concerning the nature and preparation of these epoxy novolac resins can be obtained in H. Lee and K. Neville, "Handbook of Epoxy Resins".

The amount of curing agent that is employed in curing polyepoxide compositions will depend on the amine equivalent weight of the curing agent employed. The total number of equivalents of amine group is preferably from 0.8 to 1.2 times the number of epoxide equivalents present in the curable epoxy resin composition with a stoichiometric amount being most preferred.

Various conventionally employed additives can be mixed with these polyepoxide-containing compositions before final cure. For example, in certain instances it may be desired to add minor amounts of other co-catalysts, or hardeners, along with the curing agent system herein described. Conventional pigments, dyes, fillers, flame retarding agents and other compatible natural and synthetic resins can also be added. Furthermore, known solvents for the polyepoxide materials such as acetone, methyl ethyl ketone, toluene, benzene, xylene, dioxane, methyl isobutyl ketone, dimethylformammide and ethylene glycol monoethyl ether acetate, can be used if desired, or where necessary.

The invention will be further illustrated by the following specific Examples.

Example 1

A 1-litre, three-necked flask equipped with a thermometer, a dropping funnel, a stirrer and nitrogen-inlet line was charged with p-nonylphenol (220q, 1.0M) and 116g, 0.5 mol of N,N'-diisopropyl triethylene glycol diamine. Then, formalin (37%, 81g, 1.0M) was added dropwise at ca. 30° C. over one hour. The mixture was heated to 80° C. and held at this temperature for 4.0 hours and at 110° C for two hours. During the process the generated water was removed through a Dean-Stark trap. The rest of the water was removed at 110° C and less than 0.2 KPa pressure. The product was a viscous liquid. The analysis of total amine was 2.89 meq/g (theoretical 2.81 meq/g). The viscosity was 9600cs/100° C. The NMR spectrum indicates the main product to be

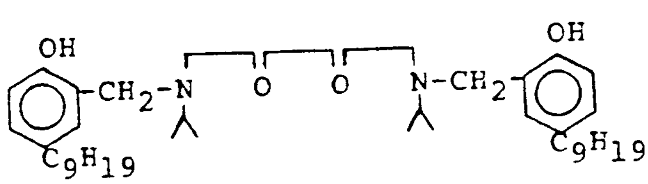

Example 2

The product of Example 1, 6622-33 (14 g.) was heated with 37% formalin (3.2g) and 22 grams of the N,N'-diisopropyl derivative of a polyoxypropylene diamine (Jeffamine $^{R}$ D-400) for four hours at 83-88 ° C., then for one hour and finally for one hour at 120 ° C and 40Pa pressure. About 80% of the product was believed to have the formula:

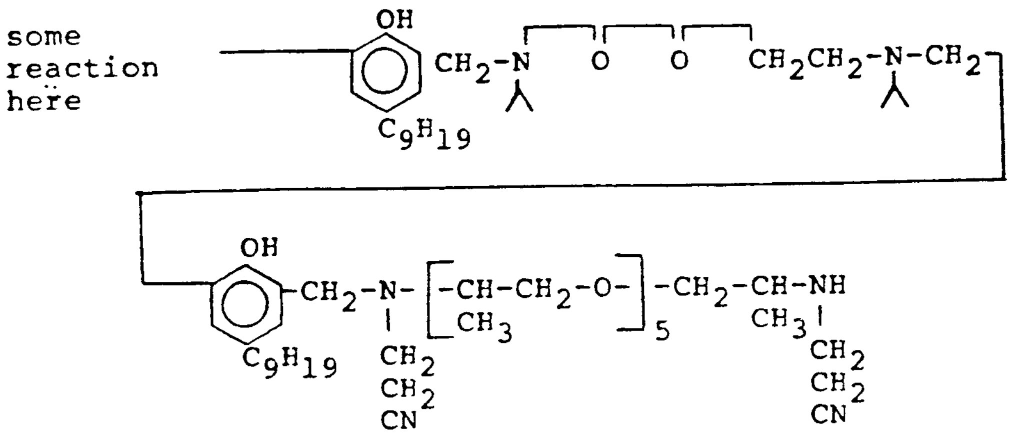


Example 3

When 2 moles of formalin and 2 moles of p-nonyl-phenol were reacted with one mole of the bis-cyanoethyl ether of Jeffamine$^{R}$-D-400 at 122 ° C., and the water of reaction was removed, the expected bis-phenol product was produced, i.e.:

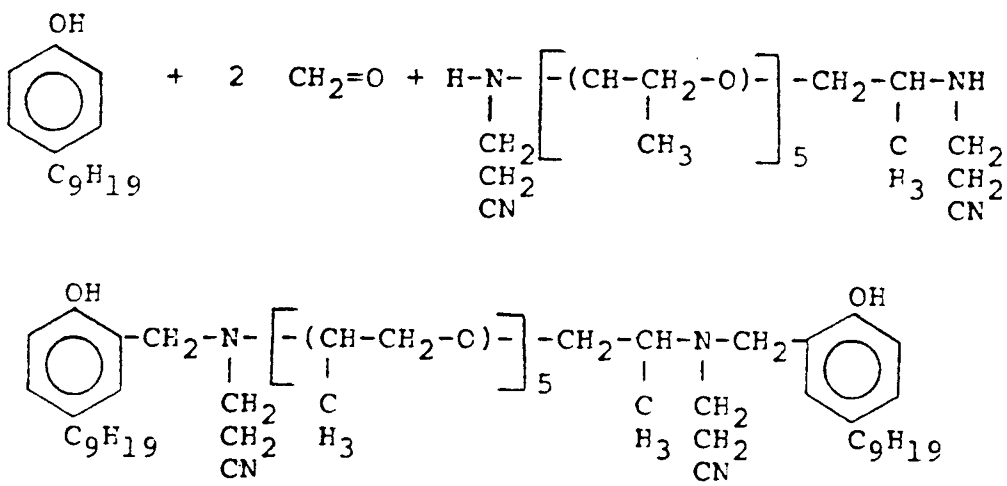

Example 4 (Comparative Example based on Example 3).

When the product of Example 3 was heated at 120 ° C. with two moles of triethyleneglycol diamine (Jeffamine$^{R}$ EDR-148 amine), and two moles formaldehyde, the product was cleaved and the Mannich reaction product Jeffamine$^{R}$ EDR-148 amine, formaldehyde and p-nonyl-phenol was formed along with some novolaks.

Example 5

The apparatus used in Example 1 was charged with nonylphenol (220g, 1.0M) and N,N'-diisopropyl EDR-148 (116g, 0.5M). Then, formalin (37%, 81g, 1.0 mole) was added dropwise at ca. 25 ° C. The exothermic temperature was reduced by an ice/water bath.

The addition process lasted about 40 minutes. The mixture was heated and kept at 85-90 ° C for 4

hours. The reaction temperature was raised to 110 ° C and water was removed through the Dean-Stark trap. At 110 ° C the mixture was subjected to a vacuum to remove trace amounts of water. The product was recovered (347g) as transparent, light-colored, viscous liquid. The total amine content was 2.89 meq/g (calc. 2.86 meq/g and viscosity was 9600 cs/100 ° C.

The H'nmr indicated the Structure A

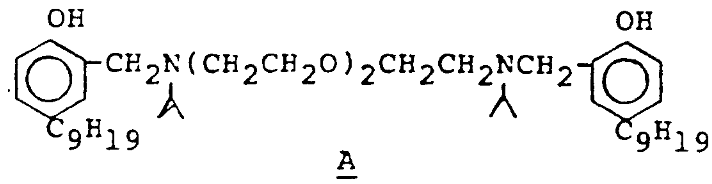

A

Example 6 (Comparative)

The experimental procedures of Example 5 were repeated, except for using phenol (94g, 1 mole), formalin (37%, 81g, 1 mole) and N, N'-diisopropyl EDR-148 (116g, 0.5M). The resulting product was a viscous liquid.

Example 7 (Comparative)

A 250 ml three-necked flask equipped with a thermometer, Dean-Stark trap, dropping funnel, stirrer and nitrogen line was charged with the product of Example 6 (0.12M) and EDR-148 (36g, 0.24 mole). Then, formalin (19g, 0.24 mole) was added dropwise over 10 minutes. The mixture was heated at 80 ° C for four hours, and water was then removed at 110-130 ° C. The product was gelled material. It is demonstrated that the corresponding phenol product cannot be made under these conditions.

Examples 8 and 9

A two-step synthesis involving different ratios of starting materials is used to make intermediate (B) and the final product (C), as illustrated by

(B)

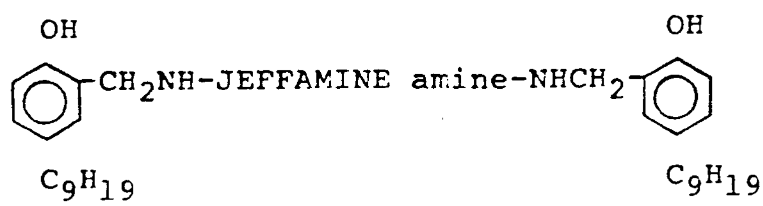

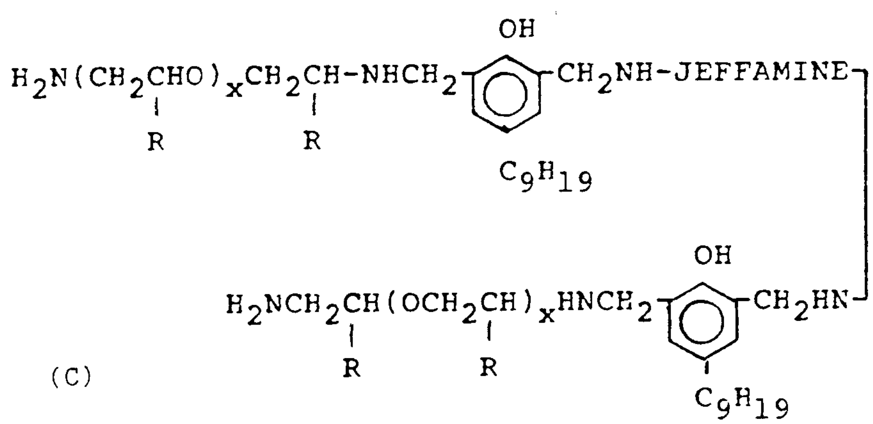

(C)

The examples of preparing (B) are cited in Example 8 and Table 1 and of preparing (C) in Example 9

and Table 2.

Example 8

The apparatus used in Example 1 was charged with p-nonylphenol (330g, 1.5M) and JEFFAMINE EDR-148 (111g, 0.75M). Then, formalin (37%, 122g, 1.5M) was added dropwise at Ca. 50 ° C over 1.5 hours. During the addition, a slurry of a white solid was observed. The mixture was slowly heated at 100-130 ° C for 4.5 hours. During the process the generated water was removed through the Dean-Stark trap. The product was recovered (439g) as a transparent, light-colored liquid. The analysis of total amine was 3.4 meq/g (theoretical 3.3 meq/g). The viscosity was 4,000 cs/66 ° C. The NMR spectrum indicates the main product to be

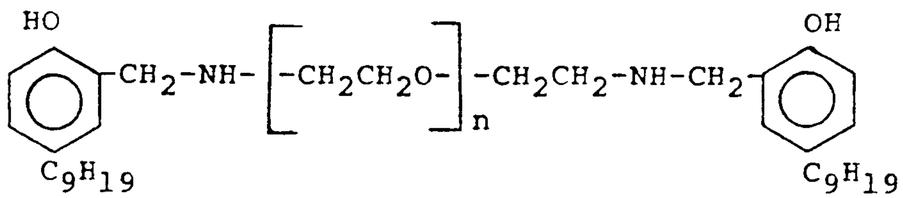

Example 9

A 500 ml, three-necked flask equipped with a thermometer, a Dean-Stark trap, a stirrer and nitrogen-inlet line was charged with the product from Example 8, (123g, ca. 0.2M) and EDR-148 (59.2g, 0.4M). Then, formalin (37%, 32.4g, 0.4M) was added dropwise under 80 ° C. The mixture was heated at 90-110 ° C for over 4 hours to remove water. The resulting product contained 6.5 meq/g total amine (6.4 meq/g calcd) and had a viscosity of 12,000 cs/37.8 ° C. The product was a light-colored liquid.

In Table 1 the various intermediates (B) were prepared according to Example 8. The structures contain a secondary amine and two nonylphenol functionalities.

In Table 2, the intermediate (B) was used and the active amine terminated Mannich products were prepared according to Example 9. These compounds contain both primary and secondary amine groups.

Example 10 Usage of Product

The product of Example 9 (11.5g) was mixed with EPON$^R$ 828 (18.7g), poured into a mould and cured at 100 ° C overnight to give a rigid, hard material.

Following the procedure of Example 8, a series of intermediate Mannich reaction products was prepared. The products prepared and the properties thereof are shown in Table 1.

TABLE 1

**SYNTHESIS OF INTERMEDIATE (B)**

| Sample | Moles | | | Product (A) | | |
|---|---|---|---|---|---|---|
| | Nonyl-phenol | Formal-dehyde | Amine | Amine meq/g | (calc) | Description |
| 8A | 2 | 2 | EDR-148 / 1 | 3.4 | (3.3) | Light coloured liq. 4,000 cs/60°C. |
| 8B | 2 | 2 | EDR-192 / 1 | 3.0 | (3.0) | Light coloured liq. 2,000 cs/66°C. |
| 8C | 2 | 2 | D-230 / 1 | 2.7 | (2.9) | Brown liquid 22,000 cs/50°C. |
| 8D | 2 | 2 | D-400 / 1 | 2.1 | (2.3) | Brown liquid 4,000 cs/50°C. |

In like manner, a series of final Mannich condensates were prepared following the procedure of Example 9. The starting materials used and the products obtained are set forth in Table 2.

TABLE 2

SYNTHESIS OF PRODUCT (C)

| Notebook No. | Moles: Intermediate | Moles: Formal-dehyde | Moles: Amine | Product (B): Amine meq/g | Product (B): (calc) | Product (B): Description |
|---|---|---|---|---|---|---|
| | (8A) | | (EDR-148) | | | |
| 9A | 1 | 2 | 2 | 6.5 | (6.4) | Light coloured liq. 12,000 cs/37.8°C. |
| | | | (EDR-192) | | | |
| 9B | 1 | 2 | 2 | 5.8 | (5.8) | Light coloured liq. 7,000 cs/37.8°C. |
| | (8B) | | (EDR-148) | | | |
| 9C | 1 | 2 | 2 | 6.0 | (6.1) | Light yellow liq. 3,000 cs/50°C. |
| | | | (EDR-192) | | | |
| 9D | 1 | 2 | 2 | 5.5 | (5.6) | Light yellow liq. 2,000 cs/50°C. |
| | (8C) | | (EDR-148) | | | |
| 9E | 1 | 2 | 2 | 5.7 | (5.9) | Light brown liq. 5,000 cs/50°C. |
| | | | (EDR-192) | | | |
| 9F | 1 | 2 | 2 | 5.3 | (5.4) | Brown liquid 3,000 cs/50°C. |
| | (8D) | | (EDR-148) | | | |
| 9G | 1 | 2 | 2 | 4.9 | (5.0) | Brown liquid 2,000 cs/50°C. |
| | | | (EDR-192) | | | |
| 9H | 1 | 2 | 2 | 4.6 | (4.7) | Brown liquid 1,500 cs/50°C. |

Example 11 (comparative)

When Sample 8A (see Table 1) was allowed to react with two moles of formalin and two moles of diethylenetriamine for 5 hours at 85-130 ° C, 80 to 85% of the ortho positions had reacted. There was about a 50% replacement of the $-NH-CH_2CH_2O$by diethylenetriamine in the ortho position.

**Claims**

**1.** Mannich condensation products having the formula

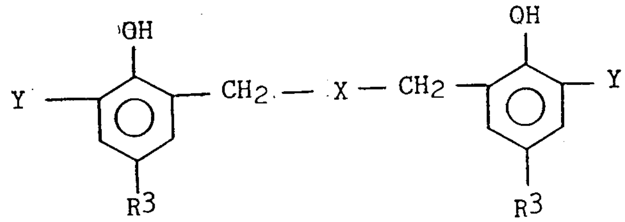

wherein
X is a group of the formula

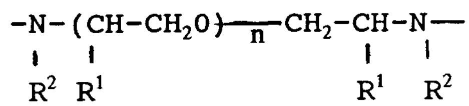

Y is hydrogen or a group of the formula

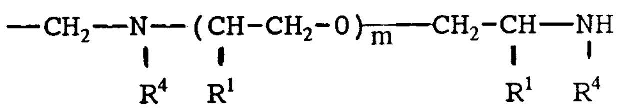

m and n are each 1-6,
$R^1$ is hydrogen or methyl
$R^2$ is hydrogen, $C_1$-$C_4$ alkyl or cyanoethyl,
$R^3$ is $C_1$-$C_{15}$ alkyl, and
$R^4$ is hydrogen, $C_1$-$C_4$ alkyl or cyanoethyl, with the proviso that when $R^2$ is not hydrogen, $R^4$ is also not hydrogen

**2.** Mannich condensation products having the formula

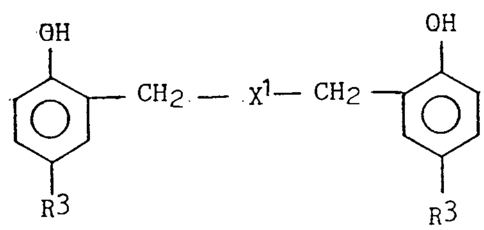

wherein
$X^1$ is a group of the formula

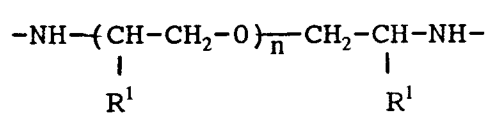

and $R^1$, $R^3$ and n have the meanings given in Claim 1.

**3.** Mannich condensation products having the formula

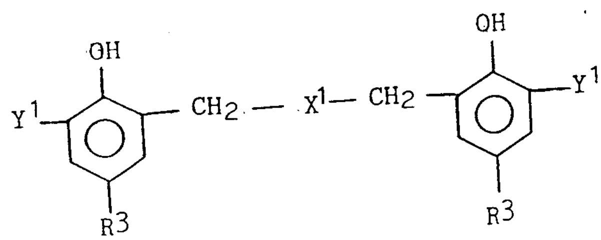

wherein
$X^1$ is a group of the formula

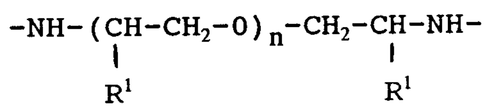

$Y^1$ is a group of the formula

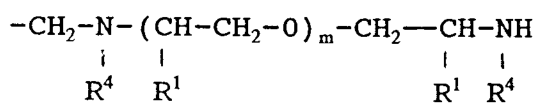

and $R^1$, $R^3$, $R^4$, m and n have the meanings given in Claim 1.

**4.** Mannich condensation products having the formula

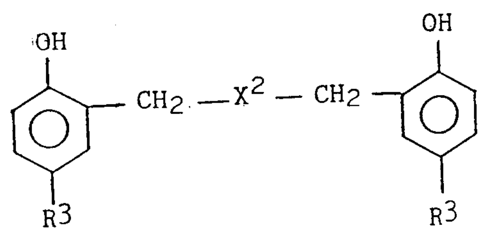

wherein
$X^2$ is a group of the formula

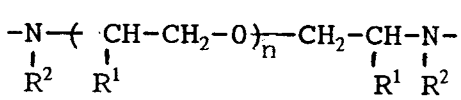

and $R^1$, $R^3$, and n have the meanings given in Claim 1, and $R^2$ is $C_1$-$C_4$ alkyl or cyanoethyl.

**5.** Mannich condensation products having the formula

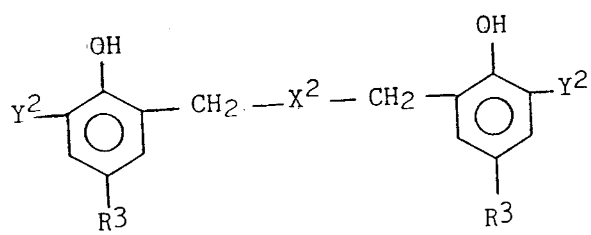

wherein
$X^2$ is a group of the formula

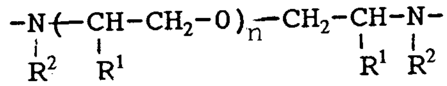

$Y^2$ is a group of the formula

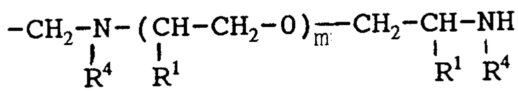

$R^1$, $R^3$, m and n have the meanings given in Claim 1, and $R^2$ and $R^4$ are $C_1$-$C_4$ alkyl or cyanoethyl.

**6.** A method for the preparation of a compound according to Claim 1 characterized by reacting a phenol of the formula

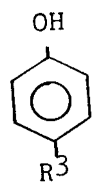

with formaldehyde and a polyoxyalkylene diamine of the formula

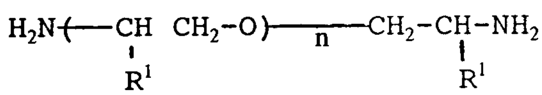

wherein $R^1$, $R^3$ and n have the meanings given in Claim 1.

**7.** A method for the preparation of a compound according to Claim 3 characterized by reacting a compound according to Claim 2 with formaldehyde and a polyoxyalkylene diamine of the formula

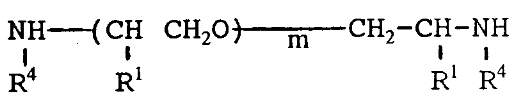

wherein $R^1$, $R^4$ and m have the meanings given in Claim 1.

**8.** A method for the preparation of a compound according to Claim 4 characterized by reacting a phenol of the formula

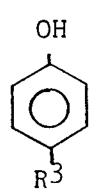

with formaldehyde and a polyoxyalkylene diamine of the formula

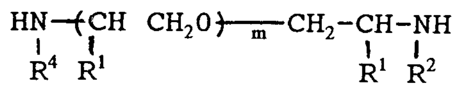

wherein $R^1$, $R^3$ and n have the meanings given in Claim 1, and $R^2$ is $C_1$-$C_4$ alkyl or cyanoethyl.

**9.** A method for the preparation of a compound according to Claim 3 characterized by reacting a compound according to Claim 2 with formaldehyde and a polyoxyalkylene diamine of the formula

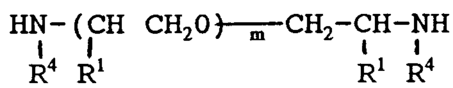

wherein $R^1$, and m have the meanings given in Claim 1, and $R^4$ is $C_1$-$C_4$ alkyl or cyanoethyl.

**10.** Use of a Mannich condensation product according to any one of Claims 1 to 5 as a curing agent for epoxy resins.

European Patent Office

# EUROPEAN SEARCH REPORT

Application Number

EP 91 30 7391

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | FR-A-2 413 411 (TEXACO DEVELOPMENT CORPORATION) * claims 1-9 * --- | 1-10 | C08G14/06 |
| Y | FR-A-2 376 166 (CHEVRON RESEARCH COMPANY) * claims 1-15 * --- | 1-10 | |
| A | EP-A-0 357 311 (TEXACO DEVELOPMENT CORPORATION) * claims 1-7 * ----- | 1-10 | |
| | | | TECHNICAL FIELDS SEARCHED (Int. Cl.5) |
| | | | C08G |

The present search report has been drawn up for all claims

| Place of search | Date of completion of the search | Examiner |
|---|---|---|
| THE HAGUE | 06 FEBRUARY 1992 | GLANDDIER A. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document